(19) Europäisches Patentamt
European Patent Office
Office européen des brevets

(11) Veröffentlichungsnummer: **0 323 625 B1**

(12) **EUROPÄISCHE PATENTSCHRIFT**

(45) Veröffentlichungstag der Patentschrift: **09.09.92**

(51) Int. Cl.5: **C07C 61/40**, C07C 51/00, C07C 49/235

(21) Anmeldenummer: **88121699.8**

(22) Anmeldetag: **27.12.88**

# (54) Verfahren zur Herstellung von 3-(2-Chlor-2(4-chlorphenyl)-vinyl)-2,2-dimethylcyclopropancarbonsäure.

(30) Priorität: **05.01.88 DE 3800097**

(43) Veröffentlichungstag der Anmeldung:
**12.07.89 Patentblatt 89/28**

(45) Bekanntmachung des Hinweises auf die Patenterteilung:
**09.09.92 Patentblatt 92/37**

(84) Benannte Vertragsstaaten:
**CH DE GB IT LI**

(56) Entgegenhaltungen:
**EP-A- 0 095 696**

(73) Patentinhaber: **BAYER AG**

**W-5090 Leverkusen 1 Bayerwerk(DE)**

(72) Erfinder: **Lantzsch, Reinhard, Dr.**
**Am Buschhäuschen 51**
**W-5600 Wuppertal 1(DE)**

EP 0 323 625 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die vorliegende Erfindung betrifft ein neues Verfahren zur Herstellung von 3-(2-Chlor-2-(4-chlorphenyl)-vinyl)-2,2-dimethylcyclopropancarbonsäure.

Es ist bekannt diese Carbonsäure durch Umsetzung von 3-Chlor-3-(4-chlorphenyl)-propenal mit Methyl-isopropylketon, Bromierung des dabei entstehenden 4,4-Dimethyl-1,3-dichlor-1-(4-chlorphenyl)-1-hexen-5-on mit Brom und anschließende Cyclisierung unter Ringverengung in Gegenwart wäßriger Laugen herzustellen (EP-OS 95 047). Die Wirtschaftlichkeit dieser Reaktionsfolge ist jedoch nicht voll befriedigend.

Es wurde nun gefunden, daß man 3-(2-Chlor-2-(4-chlorphenyl)-vinyl)-2,2-dimethylcyclopropancarbonsäure und ihre Derivate erhält, indem man in einer ersten Stufe 3-Chlor-3-(4-chlorphenyl)-propenal mit Chlormethylisopropylketon umsetzt und das entstandene 4,4-Dimethyl-1,3,6-trichlor-1-(4-chlorphenyl)-1-hexen-5-on gegebenenfalls ohne Isolierung in Gegenwart wäßriger Basen oder in Gegenwart von Alkoholaten umsetzt.

Die entstehenden Carbonsäuren bzw. Carbonsäureester lassen sich nach allgemein bekannten Methoden in ihre Derivate wir z.B. Salze, Ester, Amide, Halogenide überführen.

Durch diese Art der Reaktionsführung läßt sich die Carbonsäure in wesentlich besseren Ausbeuten erhalten als nach den bekannten Methoden. Um einen aussagekräftigen Vergleich der Ausbeuten anstellen zu können müssen folgende Verfahren zusammengefaßt und verglichen werden:

I bekannt

a) 3-Chlor-3-(4-chlorphenyl)-propenal umgesetzt mit Methylisopropylketon, Ausbeute gemäß EP-OS 95 047 Beispiel 1 = 72,7 %
b) 4,4-Dimethyl-1,3-dichlor-(4-chlorphenyl)-1-hexen-5-on umgesetzt mit Brom, Ausbeute gemäß EP-OS 95 047 Beispiel 2 (Nacharbeitung) = 75 %
c) 4,4-Dimethyl-1,3-dichlor-6-brom-(4-chlorphenyl)-1-hexen-5-onumgesetzt mit wäßrigem Alkali, Ausbeute gemäß EP-OS 95 047 Beispiel 3 (Nacharbeitung) = 82 %

Gesamtausbeute der Reaktionen a + b + c = 44,7 %.

II Vergleich

a) 3-Chlor-3-(4-chlorphenyl)-propenal umgesetzt mit Methylisopropylketon, Ausbeute gemäß EP-OS 95 047 Beispiel 1 = 72,7 %
b) 4,4-Dimethyl-1,3-dichlor-(4-chlorphenyl)-1-hexen-5-on umgesetzt mit Chlor, Ausbeute = 60,2 % (dieses Beispiel ist im Stand der Technik nicht beschrieben)
c) 4,4-Dimethyl-1,3,6-trichlor-1-(4-chlorphenyl)-1-hexen-5-on umgesetzt mit wäßrigem Alkali, Ausbeute = 94 % (dieses Beispiel ist im Stand der Technik nicht beschrieben)

Gesamtausbeute der Reaktionen a + b + c = 41,1 %.

II erfindungsgemäß

a) Methylisopropylketon umgesetzt mit Chlor, Ausbeute = 78 %
b) 3-Chlor-3-(4-chorphenyl)-propenal umgesetzt mit Chlormethylisopropylketon, Ausbeute = 92 %
c) 4,4-Dimethyl-1,3,6-trichlor-1-(4-chlorphenyl)-1-hexen-5-on umgesetzt mit mit wäßrigem Alkali, Ausbeute = 94 %

Gesamtausbeute der Reaktionen a + b + c = 67,5 %.

Durch die erfindungsgemäße Reaktionsführung wird eine Steigerung der Gesamtausbeute der gewünschten Carbonsäure von 41,1 bzw. 44,7 auf 67,5 % erreicht. Besonders überraschend war, daß in den erfindungsgemäßen Reaktionen die gewünschten Produkte in so hohen Ausbeuten erhalten werden konnten.

Die zur Durchführung der erfindungsgemäßen Reaktionen eingesetzten Ausgangsprodukte sind bekannt. Die erste Stufe der erfindungsgemäßen Reaktion kann mit oder ohne Verdünnungsmittel durchgeführt werden. Die Ausgangsprodukte werden in etwa äquimolarem Verhältnis eingesetzt. Ein Überschuß von bis zu 4 Äquivalenten Chlormethylisopropylketon, bezogen auf das 3-Chlor-3-(4-chlorphenyl)-propenal ist besonders beim Arbeiten ohne Verdünnungsmittel vorteilhaft.

Als Verdünnungsmittel komen in Frage: Kohlenwasserstoffe wie Cyclohexan, Petrolether, Benzol, Toluol, Chlorkohlenwasserstoffe wie Dichlormethan, Chloroform, Tetrachlorkohlenstoff oder Chlorbenzole, Ether wie Diethylether, Diisopropylether, Methyl-t-butylether, Tetrahydrofuran, Dioxan, 1,2-Dimethoxyethan, aliphatische Säuren wie Essigsäure, Propionsäure.

Die Reaktion wird in Gegenwart mindestens äquimolarer Mengen Chlorwasserstoff durchgeführt. Ein bis zu 4 molarer Überschuß kann von Vorteil sein.

Die Reaktion wird bei Temperaturen zwischen -20 °C und +30 °C, vorzugsweise zwischen 0 °C und +20 °C durchgeführt.

Es kann bei Normaldruck oder erhöhtem Druck gearbeitet werden.

Zur Aufarbeitung wird Lösungsmittel, überschüssiges Chlormethylisopropylketon sowie Chlorwasserstoff abdestilliert. Das so erhaltene Produkt

kann ohne weitere Reinigung in die nächste Stufe eingesetzt werden.

In der zweiten Stufe wird das in der ersten Stufe erhaltene 4,4-Dimethyl-1,3,6-trichlor-1-(4-chlorphenyl)-1-hexen-5-on in einem Verdünnungsmittel mit einer wäßrigen Base oder mit Alkoholaten umgesetzt.

Als Verdünnungsmittel kommen in Frage: Alkohole wie Methanol, Ethanol, t-Butanol. Als Basen kommen in Frage: wäßriges Alkali wie Natronlauge, Kalilauge. Als Alkoholate kommen in Frage: Natrium- oder Kaliumalkoholate wie Ethylate, Methylate.

Die Base wird vorteilhafterweise in einem Überschuß von 2 bis 10 Äquivalenten pro Äquivalent 4,4-Dimethyl-1,3,6-trichlor-1-(4-chlorphenyl)-1-hexen-5-on eingesetzt. Die Reaktionstemperatur kann in größeren Bereichen variiert werden. Üblicherweise wird bei Raumtemperatur gearbeitet.

Die Aufarbeitung des Reaktionsgemisches erfolgt in üblicher Weise durch Neutralisation, Abdestillieren des Lösungsmittels in Vakuum, Extraktion des entstandenen Esters oder Freisetzen der entstandenen Säure durch Ansäuern mit Mineralsäuren und anschließende Extraktion.

Beispiel 1 (erste Stufe der erfindungsgemäßen Reaktion)

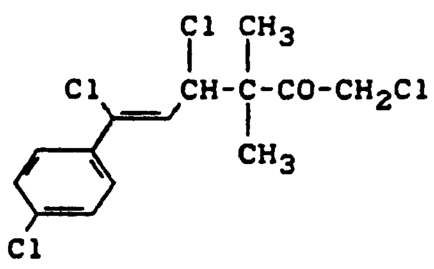

20,1 g (0,1 Mol) Z-3-Chlor-(4-chlorphenyl)-propenal und 36,2 g 1-Chlor-3-methyl-2-butanon (93 %ig = 0,28 Mol) werden vorgelegt und unter Rühren bei 10 bis 15 °C 5 Minuten Chlorwasserstoff eingeleitet. Man rührt 12 Stunden bei 20 °C nach.

Anschließend destilliert man bei 100 °C Badtemperatur/80 mbar das überschüssige Chlormethylbutanon ab.

Der Rückstand wird bei 60 °C/O,1 mbar von niedrig siedenden Nebenkomponenten befreit und kann direkt weiter umgesetzt werden: 36,4 g; Gehalt: 86 % = 92 % der Theorie. Durch Zugabe von wenig Methanol erhält man 25,8 g gelbe Kristalle vom Schmelzpunkt 72 bis 74 °C.

Beispiel 2 (zweite Stufe der erfindungsgemäßen Reaktion

Zu einer Suspension von 7 g (0,02 Mol) 4,4-Dimethyl-1,3-dichlor-6-chlor-1-(4-chlorphenyl)-1-hexen-5-on in 20 ml Methanol werden bei 20 bis 25 °C innerhalb 30 Minuten 10 g (0,11 Mol) 45 %ige Natronlauge zugetropft. Anschließend wird 3 Stunden bei 35 bis 40 °C nachgerührt. Nach der Neutralisation mit konzentrierter Salzsäure wird die Hauptmenge an Methanol im Vakuum abgedampft. Die zurückbleibende Lösung wird mit konzentrierter Salzsäure auf pH 3 gestellt mit Toluol bei 40 °C extrahiert und das Lösungsmittel abgezogen. Man erhält 5,36 g (94 % der Theorie) cis/trans-3-(Z-2-Chlor-2-(4-chlorphenyl)-vinyl-2,2-dimethyl-cyclopropancarbonsäure.

Beispiel a (Herstellung des Ausgangsprodukts)

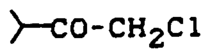

86 g (1 Mol) Methylisopropylketon und 200 ml Methanol werden vorgelegt. Dann wird unter Kühlung (stark exotherm) Chlorwasserstoff eingeleitet (ca. 4 g).

Anschließend leitet man bei -5 °C bis -10 °C 71 g (1 Mol) Chlor ein. Nach zehnminütiger Nachrührzeit wird das Gemisch aus Methanol und Chlorwasserstoff bei 25 °C/40 mbar abdestilliert (es kann für den nächsten Ansatz verwendet werden.

Der Rückstand wiegt 169,5 g und wird im Vakuum über eine 30 cm-Kolonne fraktioniert. Man erhält 94 g 1-Chlor-3-methyl-2-butanon vom Siedepunkt 73 bis 75 °C/64 mbar. Dies entspricht einer Ausbeute von 78 % Theorie.

Beispiel b) (Vergleich)

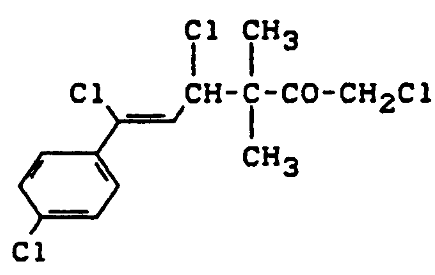

15,3 g (0,05 Mol) 4,4-Dimethyl-1,3-dichlor-1-(4-chlorphenyl)-hexen-5-on werden in 50 ml Essigsäure, die etwas Chlorwasserstoff enthält, gelöst. Unter gutem Rühren werden 3,6 g Chlor (0,05 Mol) bei 15 °C eingeleitet. Man rührt eine Stunde bei ca. 15 °C nach und engt am Dampfstrahler ein.

Man erhält 13,2 g eines Produktgemisches,

das zu 77,5 % aus 4,4-Dimethyl-1,3,6-trichlor-1-(4-chlor-phenyl)-1-hexen-5-onbesteht.
Dies entspricht einer Ausbeute von 60,2 % der Theorie.

## Patentansprüche

1. Verfahren zur Herstellung von 3-(2-Chlor-2-(4-chlorphenyl)-vinyl)-2,2-dimethylcyclopropancarbonsäure, -estern und ihren Derivaten, dadurch gekennzeichnet, daß man in einer ersten Stufe 3-Chlor-3-(4-chlorphenyl)-propenal mit Chlormethyl-isopropylketon umsetzt und in einer zweiten Stufe das entstandene 4,4-Dimethyl-1,3,6-trichlor-1-(4-chlorphenyl)-1-hexen-5-on gegebenenfalls ohne Isolierung in Gegenwart wäßriger Basen oder in Gegenwart von Alkoholaten umsetzt und gegebenenfalls anschließend die entstandene Säure oder ihre Ester in üblicher Weise derivatisiert.

## Claims

1. Process for the preparation of 3-(2-chloro-2-(4-chlorophenyl)-vinyl)-2,2-dimethylcyclopropanecarboxylic acid, its esters and its derivatives, characterised in that in a first step 3-chloro-3-(4-chlorophenyl)-propenal is reacted with chloromethyl isopropyl ketone and in a second step the resulting 4,4-dimethyl-1,3,6-trichloro-1-(4-chlorophenyl)-hex-1-en-5-one is reacted, optionally without isolation, in the presence of aqueous bases or in the presence of alkoxides, and the resulting acid or its esters is optionally subsequently derivatised in a customary manner.

## Revendications

1. Procédé de préparation de l'acide 3-[2-chloro-2-(4-chlorophényl)-vinyl]-2,2-diméthylcyclopropanecarboxylique, de ses esters et de ses dérivés, caractérisé en ce que, dans une première étape, on fait réagir le 3-chloro-3-(4-chlorophényl)-propénal avec la chlorométhylisopropylcétone et, dans une seconde étape, on fait réagir la 4,4-diméthyl-1,3,6-trichloro-1-(4-chlorophényl)-1-hexène-5-one, éventuellement sans isolement, en présence de bases aqueuses ou en présence d'alcoolates, puis éventuellement on convertit de manière habituelle l'acide formé ou ses esters en leurs dérivés.